(19) Europäisches Patentamt
European Patent Office
Office européen des brevets

(11) Veröffentlichungsnummer : **0 089 485**
**B1**

(12) # EUROPÄISCHE PATENTSCHRIFT

(45) Veröffentlichungstag der Patentschrift : **10.09.86**

(51) Int. Cl.$^4$ : **C 07 D257/04**, C 07 C119/02

(21) Anmeldenummer : **83101383.4**

(22) Anmeldetag : **14.02.83**

(54) **Verfahren zur Herstellung von 5-Chlor-1H-tetrazol-1-carbonsäureestern sowie Verfahren zur Herstellung der erforderlichen Dichlorisonitril-carbonsäureester.**

(30) Priorität : **20.03.82 DE 3210296**
**20.03.82 DE 3210297**

(43) Veröffentlichungstag der Anmeldung : **28.09.83 Patentblatt 83/39**

(45) Bekanntmachung des Hinweises auf die Patenterteilung : **10.09.86 Patentblatt 86/37**

(84) Benannte Vertragsstaaten : **BE CH DE FR GB IT LI NL SE**

(56) Entgegenhaltungen :
**DE-A- 2 500 840**
**US-A- 4 301 282**
**JOURNAL OF ORGANIC CHEMISTRY, Band 32, November 1967, Easton J.C. KAUER et al. "1-Aryltetrazoles. Synthesis and properties", Seiten 3580-3592**
**CANADIAN JOURNAL OF CHEMISTRY, Band 47, 1969, Ottawa R.RAAP et al. "Tetrazolylacetic acids", Seiten 813-819**
**ANGEWANDTE CHEMIE, Band 77, Nr. 8, 21. April 1965 I. UGI et al. "Isonitril-Synthesen", Seiten 492-504**
**ANGEWANDTE CHEMIE, Band 79, Nr. 13, 7. Juli 1967 E. KÜHLE et al. "Isocyaniddihalogenid-Synthesen" Seiten 663-680**

(73) Patentinhaber : **DYNAMIT NOBEL AKTIENGESELLSCHAFT**
**Postfach 1209**
**D-5210 Troisdorf, Bez. Köln (DE)**

(72) Erfinder : **Bison, Günter**
**Kleiststrasse 6**
**D-5210 Troisdorf (DE)**
Erfinder : **Linkat, Norbert**
**Magnolienweg**
**D-5205 St.Augustin 1 (DE)**
Erfinder : **Thewalt, Klaus, Dr.**
**Am Tiemen 12**
**D-5810 Witten (DE)**

**EP 0 089 485 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Herstellung von 5-Chlor-1H-tetrazol-1-carbonsäureestern. Verfahren zur Herstellung der genannten Verbindung sind bekannt (Can. J. Chem. 1969, *47* (5), 713-19 ; US-PS 3 468 874). Diese Verfahren verwenden in 5-Stellung mit Alkyl, Alkoxy, Amin, Halogen, Mercapto etc. substituierte Tetrazole als Ausgangsmaterial, die nicht einfach zugänglich und in der Herstellung gefährlich sind. Die anschließende Umsetzung mit Halogencarbonsäureestern führt zu einem Isomerengemisch, in dem die Carbonsäureester-Gruppe an verschiedenen Positionen des Ringes auftritt.

Aufgabe der vorliegenden Erfindung ist daher die Schaffung eines Verfahrens, welches eine technisch einfache und für die Sicherheit unbedenkliche Herstellung von 5-Chlor-1H-tetrazol-1-carbonsäureester in hoher Reinheit ermöglicht.

Gegenstand der Erfindung ist ein Verfahren zur Herstellung von 5-Chlor-1H-Tetrazol-1-carbonsäureestern der allgemeinen Formel

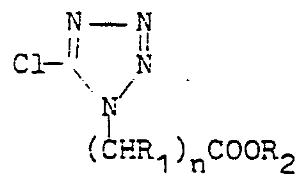

(I)

worin $R_1$ einen Alkylrest mit 1 bis 2 Kohlenstoffatomen oder Wasserstoff, n die Zahlen 1 bis 4 und $R_2$ einen Alkylrest mit 1 bis 6 Kohlenstoffatomen bedeuten, dadurch gekennzeichnet, daß der entsprechend substituierte Dichlorisonitrilcarbonsäureester der Formel

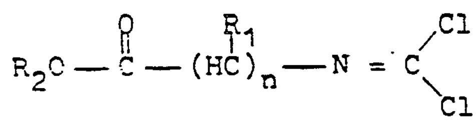

(II)

worin $R_1$, n und $R_2$ die genannte Bedeutung haben, mit einem Metallazid der Formel $MeN_3$, worin Me = Kalium, Natrium oder Ammonium bedeutet, umgesetzt und das Reaktionsprodukt isoliert wird.

Die substituierten Dichlorisonitrilcarbonsäureester sind Abkömmlinge von aliphatischen Aminocarbonsäureestern, d. h. des Aminoessigsäureesters und seiner Homologen.

Die im Produkt und Ausgangsstoff enthaltene Gruppe $—(CHR_1)_n—$ kann an einem oder mehreren der C-Atome den Substituenten $R_1$ im Sinne $—CH_3$ oder $C_2H_5$ aufweisen, von denen $—CH_3$ bevorzugt ist. Bevorzugt bedeutet n = 1 und die Gruppe denn $—CH_2—$, oder n = 2 und die Gruppe dann $H_3C—CH_2—$ oder $—CH_2—CH_2—$, für bestimmte Fälle auch n = 3 und die Gruppe dann bevorzugt $—CH_2—CH_2—CH_2—$.

Die Umsetzung kann bei Temperaturen von 10 bis 100, vorzugsweise 30 bis 90 °C, sehr bevorzugt bei der Siedetemperatur des Reaktionsgemisches im Bereich von 50 bis 90 °C durchgeführt werden.

Bevorzugt werden äquimolare Mengen der Dichlorverbindung der Formel II und des Alkaliazids eingesetzt.

Der Dichlorisonitrilcarbonsäureester der Formel II kann nach bekannten Verfahren, insbesondere aus N-Formyl-aminocarbonssäureestern der Formel

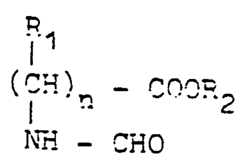

(III)

worin $R_1$, n und $R_2$ die gleiche Bedeutung haben, durch Reaktion mit Chlor oder chlorabspaltenden Verbindungen in Thionylchlorid erhalten werden.

Die Umsetzung der Ausgangsstoffe der Formel II mit einem Alkalimetallazid in Wasser oder einem Wasser enthaltenden Verdünnungsmittel wie beispielsweise Wasser/Dimethoxyäthan, Wasser/Aceton oder Wasser führt in nahezu quantitativer Ausbeute zu den Produkten der Formel I.

Die Herstellung von Tetrazolen mit Arylsubstituenten in 5-Stellung wird in J. Org. Chem. *32*, (1967), 3 580/92 aus den stabilen Arylisocyaniddichloriden durch Umsetzung mit Natriumazid beschrieben. Es war überraschend, daß gemäß der Erfindung die viel labileren Dichlorisonitrilcarbonsäureester der Formel II dieser Reaktion zugängig sind und erheblich bessere, nämlich nahezu quantitative Ausbeuten ergeben. Überraschend erfolgt weder Hydrolyse des Chloratoms in 5-Stellung oder der Carbonester-Gruppe in 1-Stellung, noch deren Substitution durch eine Azidogruppe.

Die Umsetzung der Stoffe der Formel II erfolgt bevorzugt in inerten Lösungs- oder Verdünnungsmitteln, zweckmäßig durch Eintropfen in Wasser oder einer Mischung aus Wasser und einem inerten organischen Lösungs- oder Verdünnungsmittel, das das Metallazid gelöst enthält. Geeignete organische Lösungs- oder Verdünnungsmittel sind Äther, wie Dimethoxyethan, Ketone, wie Aceton.

Die bei tiefen Temperaturen entstehenden, sehr zersetzlichen Monoazide bzw. Diazide entstehen nach unserer Verfahrensweise nicht.

Nach der Umsetzung wird aus dem Reaktionsgemisch, beispielsweise nach Abdestillieren des organischen Verdünnungsmittels und Abkühlen der verbliebenen Wasserphase auf 10 °C, das Produkt kristallisiert. Man erhält ohne weitere Reinigung 5-Chlor-tetrazol-1-carbonsäureester in hervorragend reiner Form und in hohen Ausbeuten.

Die verfahrensgemäß hergestellten Produkte lassen sich nach Austausch des in 5-Stellung vorhandenen Chloratoms gegen anderweitige Substituenten als Seitenketten für den Aufbau von Penicillinen und Cephalosporinen einsetzen und sind somit wertvolle Zwischenprodukte. Das Chloratom kann nach bekannten Methoden beispielsweise durch andere Substituenten wie Hydroxyl, Nitril, Amin, Alkoxyl oder Mercapto, sowie deren Alkylierungsprodukte ($—S—CH_2—COOR$, —SR, —SAc) ersetzt werden.

Weiterer Gegenstand der Erfindung ist ein Verfahren zur Herstellung der hierzu erforderlichen Dichlorisonitrilcarbonsäureestern d. h. von Isocyandidihalogeniden aliphatischer Carbonsäureester.

Es sind zahlreiche Methoden zu deren Herstellung bekannt, wonach beispielsweise Salze von monosubstituierten Dithiocarbamidsäuren in die Senföle überführt, anschließend chloriert, oder deren Alkyl-Cycloalyl- oder Arylderivate durch Chlorierung zu Isocyaniddichloriden umgesetzt werden (vgl. Houben-Weyl, Methoden der organischen Chemie, 4. Auflage, Bd X, S. 869-873 ; Chem. Berichte, 7, S. 1228, 1874 ; Annalen der Chemie, 663, S. 46, (1963) ; DE-AS 1 221 213).

Weitere Methoden der Herstellung bestehen in der Halogenaddition an Isonitrile oder in der Chlorierung monosubstituierter Formanilide (Angew. Chemie *79* (1967) Nr. 15 S. 663-80 und Angew. Chemie *74* (1962) Nr. 21 S. 861-67).

Diese Methoden eignen sich je nach Ausgangsmaterial sehr unterschiedlich. Soweit monosubstituierte Formamide als Ausgangsmaterial dienen, sind nach der Literatur vorwiegend aromatische Formamide angewandt. Lösungsmittel ist dann Thionylchlorid $SOCl_2$ und die chlorabspaltende Verbindung Sulfurylchlorid $SO_2Cl_2$. Die Ausbeuten sind nur dann gut, wenn der aromatische Kern infolge schon vorhandener Substituenten wie Halogen, Nitro-, Carboxyl- und Arylreste nicht ebenfalls chlorierbar ist. Substituenten, die mit Thionylchlorid zu reagieren vermögen, beispielsweise Carboxylgruppen und Carbonsäureester ergeben die nicht erwünschten Carbonsäurechloride (Angew. Chem. *74* (1962) S. 863 und Angew. Chem. *79* (1967) S. 667). Aliphatische und cycloaliphatische Formamide reagieren vorwiegend zu Isocyanaten, so daß beispielsweise N-Cyclohexylformamid nur in Ausbeuten höchstens zu 60 % Cyclohexylisocyaniddichlorid bildet.

Formamidderivate aliphatischer Carbonsäureester erschienen daher zur Herstellung von Isocyanidchloriden wenig geeignet.

Aufgabe der vorliegenden Erfindung ist daher die Schaffung eines Verfahrens, welches eine technisch einfache Herstellung von Dichlorisonitrilcarbonsäureestern in hoher Ausbeute und Reinheit ermöglicht.

Gegenstand der Erfindung ist ein Verfahren zur Herstellung substituierter Dichlorisonitril-carbonsäureester der Formel

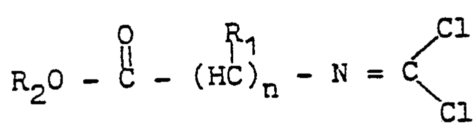

(II)

worin $R_1$ einen Alkylrest mit 1 bis 2 Kohlenstoffatomen oder Wasserstoff, n die Zahlen 1 bis 4 und $R_2$ einen Alkylrest mit 1 bis 6 Kohlenstoffatomen bedeuten, dadurch gekennzeichnet, daß N-Formylaminocarbonsäureester der Formel

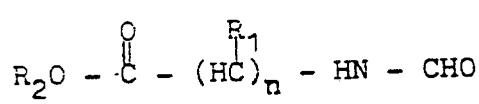

(III)

in Thionylchlorid als Lösungsmittel mit Chlor oder einer chlorabspaltenden Verbindung umgesetzt werden.

Die substituierten Dichlorisonitrilcarbonsäureester sind Abkömmlinge von aliphatischen Monocarbonsäureestern, d. h. der Essigsäureester und seiner Homologen. Die N-Formylaminocarbonsäureester haben den Typus von substituierten Formamiden.

Die im Produkt und Ausgangsstoff enthaltene Gruppe $—(CHR_1)_n—$ kann an einem oder mehreren C-Atomen den Substituenten $R_1$ im Sinne von $—CH_3$ oder $—C_2H_5$ aufweisen .

Bevorzugt bedeutet n = 1 und die Gruppe somit $—CH_2—$ oder n = 2 und die Gruppe dann $H_3C—CH_2—$ oder $—CH_2—CH_2—$, für bestimmte Fälle auch n = 3 und die Gruppe dann bevorzugt $—CH_2—CH_2—CH_2—$.

Im Gegensatz zu der genannten Literatur ist es überraschend möglich, aus den Formylderivaten der aliphatischen Aminocarbonsäureester die entsprechenden Dichlorisonitrile ohne wesentliche Anteile von Isocyanaten oder Säurechloriden in Ausbeuten von über 90 % der Theorie und hoher Reinheit zu erhalten.

Die Ausgangsstoffe der Formel III bilden sich z. B. in einfacher Weise durch Umsetzung von Aminocarbonsäureesterhydrochloriden mit Formamid in Toluol in quantitativer Ausbeute.

Die Umsetzung zu II wird vorzugsweise mit $SO_2Cl_2$ als chlorabspaltende Verbindung durchgeführt.

Das Molverhältnis der N-Formyl-aminocarbonsäureester zu Chlor bzw. der chlorabspaltenden Verbindung beträgt 1 : 1 bis 1 : 1,2. Nach beendeter Umsetzung wird das als Lösungsmittel dienende Thionylchlorid abgezogen und der Rückstand unter vermindertem Druck destilliert. Die Temperaturen können zwischen 20 und 85 °C liegen. Die Reaktion wird vorzugsweise bei Normaldruck ausgeführt. Die Zugabe der chlorabspaltenden Verbindung, z. B. $SO_2Cl_2$, zu den Stoffen der Formel III ist möglich, ergibt aber vergleichsweise geringe Ausbeuten im Bereich von 64 bis 69 % (Beispiel 4 und 5).

Vorzugsweise wird die Reaktion bei der Siedetemperatur des Thionylchlorids $SOCl_2$ durchgeführt.

Sehr hohe Ausbeuten bis zu 95 % sowie hohe Reinheiten der Produkte der Formel II werden durch eine bevorzugte Verfahrensweise dadurch erhalten, daß die chlorabgebende Verbindung, insbesondere $SO_2Cl_2$, und der Ausgangsstoff der Formel III gleichzeitig in siedendes Thionylchlorid zugegeben wird. Dabei wird etwa 10 % des $SO_2Cl_2$ am Anfang dem $SOCl_2$ zugesetzt.

Die Verbindungen der Formel II dienen beispielsweise als Ausgangsmaterial zu Cyclisierungsreaktionen mit beispielsweise Metallaziden zu substituierten Tetrazolderivaten.

Weiterer Gegenstand der Erfindung sind die neuen Stoffe nach den Ansprüchen 10 bis 12.

### Beispiel A

Dichlorisonitril-essigsäure-ethylester (Ausgangsstoff)

In einem mit vier Aufsätzen versehenen 2 000 ml Rundkolben, ausgerüstet mit Rührer, Rückflußkühler und zwei Tropftrichtern, wird ein Gemisch von 1 200 ml $SOCl_2$ und 20 ml $SO_2Cl_2$ auf Siedetemperatur erwärmt und in diese Lösung gleichzeitig 232 ml (262 g = 2 Mole) N-Formylglycinethylester und 150 ml $SO_2Cl_2$ (insgesamt 200 ml = 233,4 g = 2,47 Mole) zugetropft. Danach wird die Reaktionslösung zwei Stunden bei der angegebenen Temperatur gerührt. Zur Aufarbeitung wird das $SOCl_2$ unter Normaldruck, zuletzt bei Wasserstrahlvakuum abgezogen. Die anschließende Destillation bei $Kp_{12}$ = 93 bis 96 °C ergab 348 g = 94,5 % d. Theorie Dichlorisonitril-essigsäureethylester der Dichte $n_D^{20}$ = 1, 467 5. Die GC-Reinheit betrug : 96,8 %.

Der verwendete N-Formylglycinethylester kann aus Glycinethylester-hydrochlorid und 1,1 Mol Formamid in Toluol erhalten werden.

### Beispiel 1

5-Chlor-tetrazol-1-essigsäureethylester der Formel I

In einem mit drei Aufsätzen versehenen 1 000 ml Rundkolben, ausgerüstet mit Rührer und Rückflußkühler, wird ein Gemisch von 48,75 g (0,75 Mole) Natriumazid in einer Lösung von 180 ml Wasser und 360 ml 1,2-Dimethoxyethan auf Siedetemperatur von etwa 80 °C erhitzt. In diese Lösung werden 108 ml (1,38 g = 0,75 Mole) Dichlorisonitril-essigsäure-ethylester der Formel II innerhalb einer Stunde zugetropft und danach die Reaktionslösung 1 Stunde auf Rückflußtemperatur gehalten. Zur Aufarbeitung werden etwa 330 ml Dimethoxyethan abgezogen, die im Kolben verbliebene Lösung mit 100 ml Wasser versetzt und gekühlt. Bei 40 °C wurde die Lösung angeimpft und weiter auf 15 °C gekühlt, worauf sich nahezu farblose Kristalle abscheiden, die durch Absaugen isoliert und im Vakuum über Aetzkali getrocknet werden.

Es wurden 133,2 g Produkt (93,3 % d. Theorie) vom Fp. : 56,5 °C und gaschromatographischer Reinheit von über 99 % erhalten.

### Beispiel 2

In der in Beispiel 1 genannten Apparatur wird ein Gemisch von 48,75 g (0,75 Mole) Natriumazid in 180 ml Wasser und 360 ml Aceton im Kolben vorgelegt und in diese Lösung bei 25 °C 108 ml (1,38 g = 0,75 Mole) Dichlorisonitril-essigsäure-ethylester, verdünnt mit 100 ml Aceton zugetropft und danach die Reaktionslösung 1 Stunde auf Rückflußtemperatur gehalten. Die Aufarbeitung erfolgte entsprechend Beispiel 2. Erhalten wurden 129 g Produkt (90,3 % d. Theorie) vom Fp. : 56,5 °C.

### Beispiel 3

5-Chlor-tetrazol-1-propionsäureethylester

In einem 1 000 ml Rundkolben, ausgerüstet mit Rührer, Rückflußkühler, Tropftrichter und Thermometer, wird ein Gemisch von

32,5 g (0,5 Mole) Natriumazid in
120,0 ml Wasser und
240 ml Dimethoxyäthan auf Siedetemperatur — etwa 80 °C erhitzt- und in diese Lösung
103,1 g (Gehalt 96 %ig = 0,5 Mole) Dichlorisonitrilpropionsäureäthylester innerhalb einer Stunde zugetropft und danach die Reaktionslösung 1 Stunde auf Rückflußtemperatur gehalten.

Anschließend wurde bis Kp = 91 °C weitgehendst das Dimethoxyäthan abgezogen. Beim Kühlen der wässerigen Reaktionslösung auf 10 °C schied sich eine ölige Schicht ab, die abgetrennt wurde. Die wässerige Phase wurde noch zweimal mit je 100 ml Äther ausgeschüttelt und danach verworfen. Die ölige Phase und die Ätherextrakte wurden vereinigt, über Natriumsulfat getrocknet und anschließend nach dem Filtrieren des Trockenmittels destillativ aufgearbeitet. Bei $Kp_{0,1}$ = 63 bis 6 °C wurde eine Fraktion von 92,0 g = 90 % d. Theorie erhalten.

## Beispiel 4

Dichlorisonitrilessigsäureethylester

In einem mit drei Aufsätzen versehenen 2 000 ml Rundkolben, ausgerüstet mit Rührer, Rückflußkühler und Thermometer, wird in ein auf 60 °C erwärmtes Gemisch von 1 200 ml $SOCl_2$ und 100 ml (166,7 g = 1,24 Mole) $SO_2Cl_2$ innerhalb 120 Minuten 116 ml (131 g = 1 Mol) N-Formylglycinethylester zugetropft. Anschließend wird das Reaktionsgemisch 60 Minuten lang bei 85 °C gerührt. Danach wird das nicht umgesetzte $SO_2Cl_2$ und das Lösungsmittel $SOCl_2$ zuerst bei Normaldruck, gegen Ende im Wasserstrahlvakuum und einer Sumpftemperatur von 40 bis 45 °C abgezogen.

Die anschließende Destillation des verbliebenen Rückstandes bei $Kp_{12}$ 92 bis 95 °C ergab 127,3 g = 69,2 % der Theorie des Produktes vom Berechnungsindex $n_D^{20}$ = 1,468 3.

## Beispiel 5

Der im Beispiel 4 genannten Apparatur wurden in 1 200 ml $SOCl_2$ bei 20 °C 116 ml (131 g = 1 Mol) N-Formylglycinethylester innerhalb 60 Minuten zugetropft, wobei die Temperatur auf 35 °C anstieg. Anschließend werden in 60 Minuten 200 ml (334 g = 2,48 Mole) $SO_2Cl_2$ zugetropft und die Temperatur danach auf 75 °C erhöht und 60 Minuten unter Rühren bei 75 °C gehalten. Bei Aufarbeitung wie im Beispiel 1 wurden bei $Kp_{12}$ = 90 bis 95 °C 118,5 g = 64,4 % der Theorie des Produktes $n_D^{20}$ = 1,468 5 erhalten.

## Beispiel 6

Dichlorisonitril-essigsäure-ethylester

In einem mit vier Aufsätzen versehenen 2 000 ml Rundkolben, ausgerüstet mit Rührer, Rückflußkühler und zwei Tropftrichtern, wird ein Gemisch von 1 200 ml $SOCl_2$ und 20 ml $SO_2Cl_2$ auf 75 bis 80 °C erwärmt und in diese Lösung gleichzeitig 232 ml (262 g = 2 Mole) N-Formylglycinethylester und 150 ml $SO_2Cl_2$ (zusammen 233,4 g = 2,47 Mole $SO_2Cl_2$) zugetropft. Nach beeindetem Zulauf wird die Reaktionslösung zwei Stunden bei der angegebenen Temperatur gerührt. Zur Aufarbeitung wird das $SOCl_2$ zuerst unter Normaldruck, gegen Ende der Destillation im Wasserstrahlvakuum abgezogen. Hierbei wurden 990 ml = 94,6 % d. Theorie vom eingesetzten $SOCl_2$ zurückerhalten.

Die anschließende Destillation bei $Kp_{12}$ = 93 bis 96 °C ergab 348 g Produkt = 94,5 % der Theorie als farbloses Destillat vom $n_D^{20}$ = 1,467 5. Die gaschromatographische Reinheit betrug 96,8 %.

## Beispiel 7

α-(Dichlorisonitril) propionsäureethylester

In einem mit vier Aufsätzen versehenen 1 000 ml Rundkolben ausgerüstet mit Rührer, Rückflußkühler und zwei Tropftrichtern, wird ein Gemisch von 400 ml $SOCl_2$ und 5 ml $SO_2Cl_2$ auf 65 bis 75 °C erwärmt und in diese Lösung gleichzeitig 72 g α-(N-Formylamino)-propionsäureethylester ($Kp_{20}$ 141 bis 143 °C) und 50 ml $SO_2Cl_2$ zugetropft. Nach analogem Reaktionsverlauf wie in Beispiel 3 erhält man bei der anschließenden Destillation 83 g = 88,7 d. Th. eines farblosen Destillats ($Kp_{13}$ 88 bis 90 °C, $n_D^{20}$ 1,459 5).

## Beispiel 8

β-(Dichlorisonitril) propionsäureethylester

Unter analogen Bedingungen wie Beispiel 4 werden eingesetzt
400 ml $SOCl_2$

10 ml $SO_2Cl_2$
anschließend
100 g β-(N-Formylamino)propionsäureethylester ($Kp_{18}$ 149 bis 151 °C).
Es werden erhalten 119 g = 89,6 % d. Theorie farbloses Destillat ($Kp_{13}$ 101 bis 103 °C, $n_D^{20}$ 1,464 8).

## Patentansprüche

1. Verfahren zur Herstellung von 5-Chlor-1H-tetrazol-1-carbonsäureestern der Formel

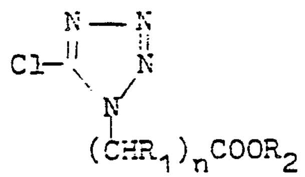

(I)

worin $R_1$ einen Alkylrest mit 1 bis 2 Kohlenstoffatomen oder Wasserstoff, n die Zahlen 1 bis 4 und $R_2$ einen Alkylrest mit 1 bis 6 Kohlenstoffatomen bedeuten, dadurch gekennzeichnet, daß der entsprechend substituierte Dichlorisonitrilcarbonsäureester der Formel

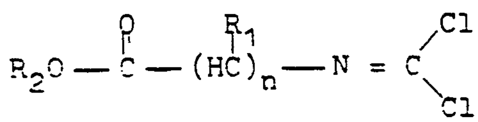

(II)

worin $R_1$, n und $R_2$ die genannte Bedeutung haben, mit einem Metallazid der Formel $MeN_3$, worin Me = Kalium, Natrium oder Ammonium bedeutet, umgesetzt und das Reaktionsprodukt isoliert wird.

2. Verfahren nach Anspruch 1, dadurch gekennzeichnet, daß die Umsetzung bei Temperaturen von 10 bis 100, vorzugsweise 50 bis 90 °C, durchgeführt wird.

3. Verfahren nach einem der Ansprüche 1 oder 2, dadurch gekennzeichnet, daß die Umsetzung bei der Siedetemperatur des verwendeten Verdünnungs- oder Lösungsmittels durchgeführt wird.

4. Verfahren nach Anspruch 3, dadurch gekennzeichnet, daß die Umsetzung in Wasser, gegebenenfalls in Mischung mit inerten Lösungs- oder Verdünnungsmitteln durchgeführt wird.

5. Verfahren nach mindestens einem der Ansprüche 1 bis 4, dadurch gekennzeichnet, daß äquivalente Mengen der Ausgangsstoffe der Formel II und der Metallazide umgesetzt werden.

6. Verfahren zur Herstellung substituierter Dichlorisonitril-carbonsäureester der Formel

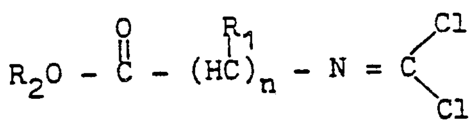

(II)

worin $R_1$ einen Alkylrest mit 1 bis 2 Kohlenstoffatomen oder Wasserstoff, n die Zahlen 1 bis 4 und $R_2$ einen Alkylrest mit 1 bis 6 Kohlenstoffatomen bedeuten, dadurch gekennzeichnet, daß N-Formylaminocarbonsäureester der Formel

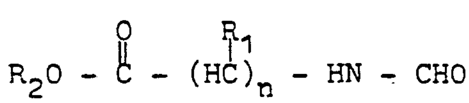

(III)

worin $R_1$, n und $R_2$ die genannte Bedeutung haben, in Thionylchlorid als Lösungsmittel mit Chlor oder einer chlorabspaltenden Verbindung umgesetzt werden.

7. Verfahren nach Anspruch 6, dadurch gekennzeichnet, daß die Umsetzung von Reaktionsbeginn an bei Siedetemperatur durchgeführt wird.

8. Verfahren nach einem der Ansprüche 6 oder 7, dadurch gekennzeichnet, daß als chlorabspaltende Verbindung Sulfurylchlorid verwendet wird.

9. Verfahren nach Anspruch 6 bis 8, dadurch gekennzeichnet, daß das Molverhältnis von N-Formylaminocarbonsäureester zu Chlor bzw. der chlorabspaltenden Verbindung 1 : 1 bis 1 : 1,2 beträgt.

10. Dichlorisonitrilessigsäureethylester.

11. Dichlorisonitril-α-methyl-essigsäureethylester.

12. Dichlorisonitrilpropionsäureethylester.

## Claims

1. Process for the production of 5-chloro-1H-tetrazol-1-carboxylic acid esters of the formula

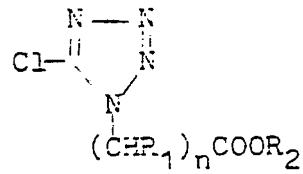

(I)

wherein $R_1$ denotes an alkyl residue with 1 to 2 carbon atoms or hydrogen, n denotes the numbers 1 to 4 and $R_2$ denotes an alkyl residue with 1 to 6 carbon atoms, characterised in that the correspondingly substituted dichloroisocyanocarboxylic acid ester of the formula

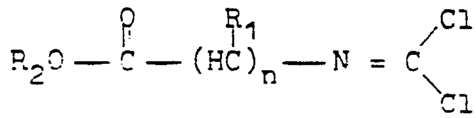

(II)

wherein $R_1$, n and $R_2$ have the indicated meaning is reacted with a metal azide of the formula $MeN_3$, wherein Me = potassium, sodium or ammonium, and the reaction product is isolated.

2. Process according to claim 1, characterised in that the reaction is carried out at temperatures of 10 to 100, preferably 50 to 90 °C.

3. Process according to one of claims 1 or 2, characterised in that the reaction is carried out at the boiling temperature of the diluent or solvent used.

4. Process according to claim 3, characterised in that the reaction is carried out in water, optionally in a mixture with inert solvents or diluents.

5. Process according to at least one of claims 1 to 4, characterised in that equivalent amounts of the starting materials of formula II and the metal azides are reacted.

6. Process for the production of substituted dichloroisocyanocarboxylic acid esters of the formula

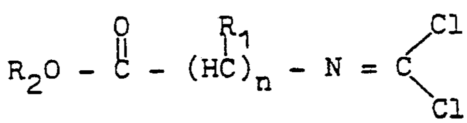

(II)

wherein $R_1$ denotes an alkyl residue with 1 to 2 carbon atoms or hydrogen, n denotes the numbers 1 to 4 and $R_2$ denotes an alkyl residue with 1 to 6 carbon atoms, characterised in that N-formylamino carboxylic acid esters of the formula

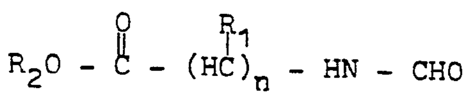

(III)

wherein $R_1$, n and $R_2$ have the indicated meaning, are reacted in thionyl chloride as solvent with chlorine or a compound from which chlorine splits off.

7. Process according to claim 6, characterised in that the reaction is carried out right from the reaction beginning at boiling temperature.

8. Process according to one of claims 6 or 7, characterised in that sulphuryl chloride is used as compound splitting off chlorine.

9. Process according to claims 6 to 8, characterised in that the molar ratio of N-formylamino carboxylic acid ester to chlorine or the compound splitting off chlorine amounts to 1 : 1 to 1 : 1.2.

10. Dichloroisocyanoacetic acid ethyl ester.

11. Dichloroisocyano-α-methyl acetic acid ethyl ester.

12. Dichloroisocyanopropionic acid ethyl ester.

## Revendications

1. Procédé pour préparer des esters d'acides 5-chloro-1H-tétrazolo-1-carboxyliques de formule :

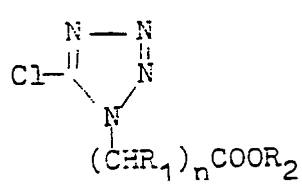

(I)

(dans laquelle $R_1$ représente un reste alkyle ayant 1 ou 2 atomes de carbone ou un àtome d'hydrogène, n

représente les nombres 1 à 4 et $R_2$ représente un reste alkyle ayant 1 à 6 atomes de carbone), caractérisé en ce qu'on fait réagir l'ester d'acide dichloroisonitrile carboxylique, substitué de façon correspondante, de formule :

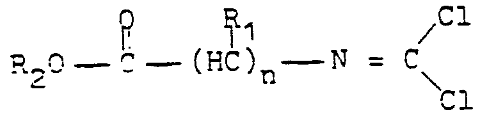

(II)

(dans laquelle $R_1$, n et $R_2$ ont le sens indiqué) avec un azoture de métal de formule $MeN_3$ (dans laquelle Me représente le potassium, le sodium ou l'ammonium) et l'on isole le produit de la réaction.

2. Procédé selon la revendication 1, caractérisé en ce qu'on conduit la réaction à des températures de 10 à 100, avantageusement de 50 à 90 °C.

3. Procédé selon l'une des revendications 1 ou 2, caractérisé en ce qu'on conduit la réaction à la température d'ébullition du diluant ou solvant utilisé.

4. Procédé selon la revendication 3, caractérisé en ce qu'on conduit la réaction dans de l'eau, éventuellement en mélange avec un solvant ou diluant inerte.

5. Procédé selon l'une au moins des revendications 1 à 4, caractérisé en ce qu'on fait réagir des quantités équivalentes des substances de départ de formule II et de l'azoture de métal.

6. Procédé pour préparer des esters d'acides dichloroisonitrile-carboxylique substitué, de formule :

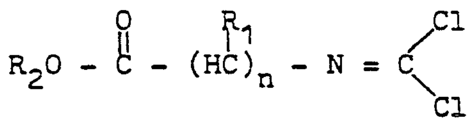

(II)

(dans laquelle $R_1$ représente un reste alkyle comportant 1 ou 2 atomes de carbone ou un atome d'hydrogène, n représente les nombres 1 à 4 et $R_2$ représente un reste alkyle ayant 1 à 6 atomes de carbone), caractérisé en ce qu'on fait réagir des esters d'acides N-formylaminocarboxyliques de formule :

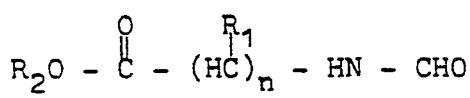

(III)

(dans laquelle $R_1$, n et $R_2$ ont le sens indiqué) dans du chlorure de thionyle comme solvant, avec du chlore ou avec un composé cédant du chlore.

7. Procédé selon la revendication 6, caractérisé en ce qu'on conduit la réaction, depuis le début de la réaction, à la température d'ébullition.

8. Procédé selon l'une des revendications 6 ou 7, caractérisé en ce qu'on utilise le chlorure de sulfuryle comme composé cédant du chlore.

9. Procédé selon l'une des revendications 6 à 8, caractérisé en ce que le rapport molaire de l'ester d'acide N-formylaminocarboxylique au chlore ou au composé cédant du chlore est de 1 : 1 à 1 : 1,2.

10. Ester éthylique de l'acide dichlorisonitrile-acétique.

11. Ester éthylique de l'acide dichlorisonitrile-alpha-méthyl-acétique.

12. Ester éthylique de l'acide dichlorisonitrile-propionique.